# EUROPEAN PATENT SPECIFICATION

(11) **EP 0 659 435 B1**
(45) Date of publication and mention of the grant of the patent: **14.05.2003**
(21) Application number: 95101919.9
(22) Date of filing: 03.12.1987
(51) Int. Cl.: A61K 31/704, A61K 9/08, A61P 35/00

(54) **Injectable ready-to-use solutions containing Doxorubicin**
Doxorubicin enthaltende einspritzbare gebrauchsfertige Lösungen gegen Krebs
Solutions injectables prêtes à l'emploi contenant Doxorubicin

(30) Priority: 05.12.1986 GB 8629193; 22.06.1987 US 64653
(43) Date of publication of application: 28.06.1995
(62) Divisional of application: 91102986.6
(73) Proprietor: Pharmacia Italia S.p.A., 20152 Milano (IT)
(72) Inventor: Gatti, Gaetano, Sesto San Giovanni, Milan (IT); Oldani, Diego, Robecco sul Naviglio, Milan (IT); Bottoni, Giuseppe, I-24100 Bergamo (IT); Confalonieri, Carlo, Cusano Milanino, Milan (IT); Gambini, Luciano, Cornaredo, Milan (IT); De Ponti, Roberto, Milan (IT)
(74) Representative: Woods, Geoffrey Corlett

(56) References cited:
- FR-A- 2 405 957
- GB-A- 2 178 311
- J.PARENT.SCI.TECHN., vol.39, no.6, 1985 pages 220 - 222 'Stability of anthracycline antitumor agents in infusion fluids'
- WINDHOLZ ET AL. 'The Merck Index, 10th ed.' 1983 , MERCK & CO , RAHWAY, US page 499, abstract no.3435: "Doxorubicin"

## Description

The present invention relates to a storage stable, injectable ready-to-use solution of doxorubicin, and to a process for preparing such a solution.

The anthracycline glycoside compounds are a well known class of compounds in the antineoplastic group of agents, of which doxorubicin is a typical, and the most widely used, representative: Doxorubicin. Anticancer Antibiotics, Federico Arcamone, 1981, Publ: Academic Press, New York, N.Y.; Adriamycin Review, EROTC International Symposium, Brussels, May, 1974, edited by M. Staquet, Publ. Eur. Press Medikon, Ghent, Belg.; and Results of Adriamycin Therapy, Adriamycin Symposium at Frankfurt/Main 1974 edited by M.Ghione, J.Fetzer and H.Maier, publ.: Springer, New York, N.Y.

At present, anthracycline glycoside antitumor drugs, in particular doxorubicin, are solely available in the form of lyophilized preparations, which need to be reconstituted before administration. Both the manufacture and the reconstitution of such preparations expose the involved personnel (workers, pharmacists, medical personnel, nurses) to risks of contamination which are particularly serious due to the toxicity of the antitumor substances.

The Martindale Extra Pharmacopoeia 28th edition, page 175 left column, reports on adverse effects of antineoplastic drugs and recommends that "They must be handled with great care and contact with skin and eyes avoided; they should not be inhaled. Care must be taken to avoid extravasation since pain and tissue damage may ensue.". Similarly, Scand. J. Work Environ Health vol.10 (2), pages 71-74 (1984), as well as articles in Chemistry Industry, Issue July 4, 1983, page 488, and Drug-Topics-Medical-Economics-Co, Issue February 7, 1983, page 99 report about severe adverse effects observed in medical personnel exposed to use of cytostatic agents, including doxorubicin.

To administer a lyophilized preparation, double handling of the drug is required. The lyophilized cake first has to be reconstituted and then administered. Moreover, in some cases, the complete dissolution of the powder may require prolonged shaking because of solubilization problems. Reconstitution of a lyophilized cake or powder can result in formation of aerosol droplets which can be inhaled or can come into contact with skin or mucous membranes of those handling the solution.

As the risks connected with the manufacture and the reconstitution of a lyophilized preparate would be highly reduced if a ready-to-use solution of the drug were available, we have developed a stable, therapeutically acceptable intravenously injectable solution of an anthracycline glycoside drug, e.g. doxorubicin, whose preparation and administration does not require either lyophilization or reconstitution.

GB-A-2178311 describes and claims sterile, pyrogen-free, anthracycline glycoside solutions which consist essentially of a physiologically acceptable salt of an anthracycline glycoside such as doxorubicin dissolved in a physiologically acceptable solvent therefor, which have not been reconstituted from a lyophilizate and which have a pH of from 2.5 to 6.5. Especially preferred pH's are about 3 and about 5. The Examples illustrate solutions with pH's ranging from 2.62 to 3.14, with pH 4.6 and with pH 5.2. There is no mention of stabilising agents. Dextrose, lactose and mannitol are mentioned as tonicity adjustment agents but no indication is given of the proportions in which they may be used.

The present invention provides a product which is a storage-stable sterile, pyrogen-free, injectable doxorubicin solution which is sealed in a container and which consists essentially of doxorubicin hydrochloride dissolved in a physiologically acceptable aqueous solvent therefor at a concentration of doxorubicin of from 0.1 to 50 mg/ml, which has not been reconstituted from a lyophilisate and the pH of which has been adjusted to from 3.5 to 4.0 solely by means of a physiologically acceptable acid.

It is thus possible to provide solutions which are storage stable and have a commercially meaningful shelf-life.

The solution of the invention is provided in a sealed container, especially one made of glass. The solution can be provided in this way either in a unit dosage form or in a multiple dosage form.

Any aqueous solvent which is physiologically acceptable and which is able to dissolve the doxorubicin salt may be used. The solution of the invention may also contain one or more formulation adjuvants such as a co-solubilising agent (which may be the same as a solvent), a tonicity adjustment agent, a preservative and a pharmaceutically acceptable chelating agent.

Suitable solvents and co-solubilising agents may be, for instance, water e.g. Water for Injections; a 0.9% sodium chloride solution, i.e. physiological saline; an aqueous 5% dextrose solution; and mixtures of water and one or more of:
- an aliphatic amide, e.g. N,N-dimethylacetamide, N-hydroxy-2-ethyllactamide and the like;
- an alcohol, e.g. ethanol, benzyl alcohol and the like;
- a glycol or polyalcohol, e.g. propyleneglycol, glycerin and the like;
- an ester of a polyalcohol, e.g. diacetine, triacetine and the like;
- a polyglycol or a polyether, e.g. polyethyleneglycol 400, propyleneglycol methylethers and the like;
- a dioxolane, e.g. isopropylidenglycerin and the like;
- dimethylisosorbide; and
- a pyrrolidone derivative, e.g. 2-pyrrolidone, N-methyl-2-pyrrolidone, polyvinylpyrrolidone and the like. Examples of preferred solvents are water, physiological saline, an aqueous
5% dextrose solution and mixtures of water and one or more of ethanol, polyethyleneglycol and dimethylacetamide.

Water, physiological saline and a 5% dextrose solution are particularly preferred.

Suitable tonicity adjustment agents may be, for instance, physiologically acceptable inorganic chlorides, e.g. sodium chloride (preferably 0.9% by weight sodium chloride), dextrose, lactose, mannitol, sorbitol and the like.

Preservatives suitable for physiological administration may be, for instance, esters of para-hydroxybenzoic acid (e.g. methyl, ethyl, propyl and butyl esters, or mixtures of them), chlorocresol and the like.

A suitable pharmaceutically acceptable chelating agent may be ethylenediaminotetraacetic acid (EDTA). The chelating agent is included in a minor amount, typically from 0.001 to 0.05% by weight.

The above mentioned solvents, tonicity adjustment agents, preservatives and chelating agents can be used alone or as a mixture of two or more of them.

To adjust the pH within the range of from 3.5 to 4.0 a physiologically acceptable acid is added. The acid may be any physiologically acceptable acid, e.g. an inorganic mineral acid such as hydrochloric, sulfuric, phosphoric and the like, or an organic acid such as acetic, succinic, tartaric, ascorbic, citric, glutamic, methanesulphonic, ethanesulfonic and the like.

Typically, the solution has a pH of from 3.7 to 4.0.

In the solutions of the invention the concentration of doxorubicin may vary from 0.1 mg/ml to 50 mg/ml, preferably from 1 mg/ml to 20 mg/ml. Other preferred concentrations for doxorubicin are from about 2 mg/ml to about 50 mg/ml, preferably from 2 mg/ml to 20 mg/ml, particularly appropriate values being 2 mg/ml and 5 mg/ml.

Suitable packaging for the doxorubicin solutions may be all approved containers intended for parenteral use, such as plastic and glass containers, ready-to-use syringes and the like. Preferably the container is a sealed glass container, e.g. a vial or an ampoule. A hermetically sealed glass vial is particularly preferred.

The invention also provides a process for producing a product according to the invention which process comprises
(i) dissolving the doxorubicin hydrochloride, which is not in the form of a lyophilisate, in the physiologically acceptable aqueous solvent therefor at a concentration of doxorubicin of from 0.1 to 50 mg/ml;
(ii) optionally, adding one or more formulation adjuvants selected from co-solubilising agents, tonicity adjustment agents, preservatives and pharmaceutically acceptable chelating agents;
(iii) adding solely a physiologically acceptable acid to adjust the pH to from 3.5 to 4.0 as desired; and
(iv) sealing the solution in a container
the process being effected in such a manner that the resultant solution is sterile and pyrogen-free.

Any suitable procedure may be adopted to ensure that the resultant solution is sterile and pyrogen-free. Preferably, the solution is passed through a sterilising filter after step (iii) but before step (iv) although of course one or more of the materials used may be sterile and pyrogen-free anyway. Where all materials employed are sterile and pyrogen-free, there may then be no need for passing the resultant solution through a sterilising filter.

With the solutions of the invention it is possible to obtain compositions having a very high concentration of the active substance even at 50 mg/ml. This constitutes a great advantage over the presently available lyophilized preparates wherein high concentrations can only be obtained with difficulty because of solubilization problems encountered in reconstitution, mainly with saline. The presence of the excipient, e.g. lactose, in the lyophilized cake, and its generally high proportion in respect of the active substance, even up to 5 parts of excipient per part of active substance, has a negative effect on solubilization so that difficulties may arise in obtaining dissolution of the lyophilized cake, especially for concentrations of doxorubicin higher than 2 mg/ml.

The solutions of the invention are characterized by a good stability. Solutions in various solvents and with different pH's and concentrations have been found to be stable for long periods at temperatures accepted for the storage of pharmaceutical preparations. This is illustrated in the Examples which follow.

Owing to the well known anti-tumor activity of the active drug substance, the pharmaceutical compositions of the invention are useful for treating tumors in both human and animal hosts. Examples of tumors that can be treated are, for instance, sarcomas, including osteogenic and soft tissue sarcomas, carcinomas, e.g., breast-, lung-, bladder-, thyroid-, prostate- and ovarian carcinoma, lymphomas, including Hodgkin and non-Hodgkin lymphomas, neuroblastoma, melanoma, myeloma, Wilms tumor, and leukemias, including acute lymphoblastic leukemia and acute myeloblastic leukemia.

Examples of specific tumours that can be treated are Moloney Sarcoma Virus, Sarcoma 180 Ascites, solid Sarcoma 180, gross transplantable leukemia, L 1210 leukemia and lymphocytic P 388 leukemia.

Inhibition of the growth of a tumour, in particular one of those indicated above, can be achieved by administering to a host suffering from a said tumour an injectable solution according to the invention containing the active drug substance in an amount sufficient to inhibit the growth of said tumour.

The injectable solutions of the invention are administered by rapid e.g. intravenous injection or infusion according to a variety of possible dose schedules.

A Suitable dose schedule for doxorubicin may be, for example, of 60 to 75 mg of active drug substance per m² of body surface given as a single rapid infusion and repeated at 21 days. An alternative schedule may be of 30 mg/m² day be intravenous route for 3 days, every 28 days.

The following Examples illustrate the invention.

### Example 1: Long term stability of doxorubicin formulations having a pH of 3.5

Batches tested, formulations tested and packaging used are shown in Tables 1 to 8. The formulations were prepared as follows.

Doxorubicin.HCl was dissolved in about 90 percent of the stated amount of water for injections or physiological saline, deaerated by nitrogen bubbling. Hydrochloric acid was added dropwise to adjust the pH of the solution to 3.5. Deaerated water for injections or physiological saline was then added in order to obtain a doxorubicin.HCl concentration of 2 mg/ml. The solutions were filtered through a 0.22 µm microporous membrane under nitrogen pressure. Volumes of 5 ml of the solutions were distributed into type I, colourless glass vials having 8 ml top capacity, respectively. The vials were then closed with chlorobutyl teflon-faced rubber stoppers and sealed with aluminium caps. The formulations thus prepared were tested as regards appearance, clarity of solutions, pH, sterility (8°C, yearly), doxorubicin.HCl assay.

### Test methods

For appearance and clarity: visual inspection
- For pH:: USP XXI
- For sterility:: USP XXI (membrane filtration)
- For doxorubicin.HCl assay:: HPLC ion-pair method and USP HPLC method (USP XXI)
Brief description of the HPLC ion-pair method for doxorubicin.HCl assay:
- Column filling :: reverse phase, Zorbax TMS
- Mobile phase :: water: acetonitrile: methanol (54:29:17 v/v/v) containing 2 ml/l 85% phosphoric acid and 1 mg/ml sodium laurylsulfate (pairing agent) adjusted to pH 3.5 with 2N NaOH
- Mobile phase flow rate :: 1.5 ml/min
- Column temperature :: ambient (22°C ± 2°C)
- Analytical wavelength :: 254 nm
- System suitability parameters :: symmetry factor between 0.7 an 1.2; number of theoretical plates ≥ 2500; measurement reproducibility: variation coefficient <1, n=6; resolution factor ≥ 12

The HPLC ion-pair method for doxorubicin.HCl assay is validated for accuracy, precision, linearity, sensitivity, specificity and stability-indicating nature.

The results obtained for:
. percent doxorubicin.HCl stability (ion-pair method) and
   .. pH
referred to the vials stored in upright position are given in:
Table 4 storage at + 4°C
Table 5 storage at + 8°C
Table 6 storage at +15°C
Table 7 storage at +27°C
Table 8 storage at 100 and 250 foot candles

The doxorubicin.HCl assays given in these Tables are the mean of three independent determinations. Also, the stabilizing effect of various agents is shown in Table 26.

As far as the other parameters checked during stability:
- the clarity of the solution was unchanged at all the checks carried out at all the storage conditions applied
- the appearance of the solutions was: a) unchanged at all the checks carried out on samples stored at 4°C and 8°C, b) slightly darkened after: 9 months at 15°C, 3 months at 27°C, 3 months at 100 and 250 foot candles light
- the closure system was unchanged at all the checks carried out at all the storage conditions
- the sterility was maintained after 12 months at 8°C.

The results of the controls carried out on the vials stored in the inverted position did not differ significantly from those on the vials in the upright position.

The percent doxorubicin.HCl stability values obtained by the USP HPLC method did not differ significantly from those obtained by the HPLC ion-pair method given in Tables 4-8.

The obtained stability data indicate that the tested doxorubicin.HCl solutions reach the lower limit of acceptance (90% of nominal concentration) in about 9 and 2-3 months at 15°C and, respectively, 27°C, but prove stable up to 12 months at 4°C and 8°C, i.e. at the temperature usually adopted for the storage of the products under refrigeration. This is in contrast to the doxorubicin.HCl solutions obtained upon reconstitution of the commercial freeze-dried preparate, whose pHs vary between 4.5 and 6 and which show a much lower stability. Indeed, it is recommended that reconstituted solutions should be discarded after only 48 hours storage in a refrigerator.

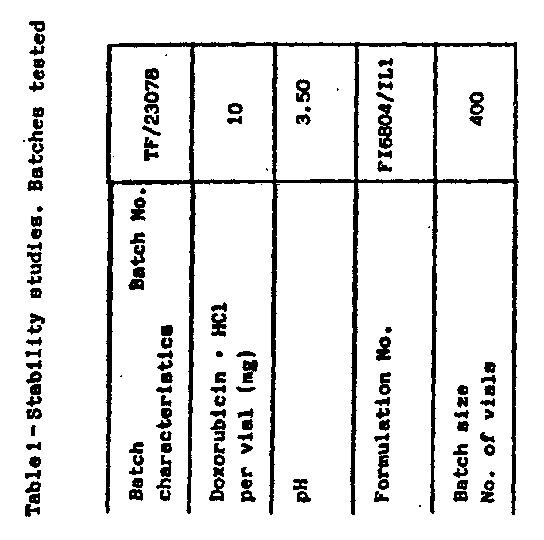

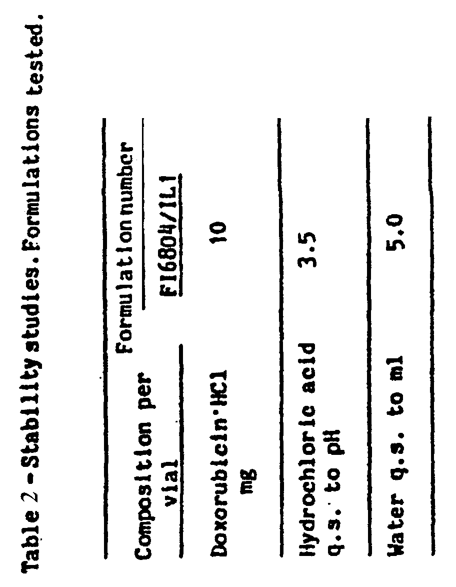

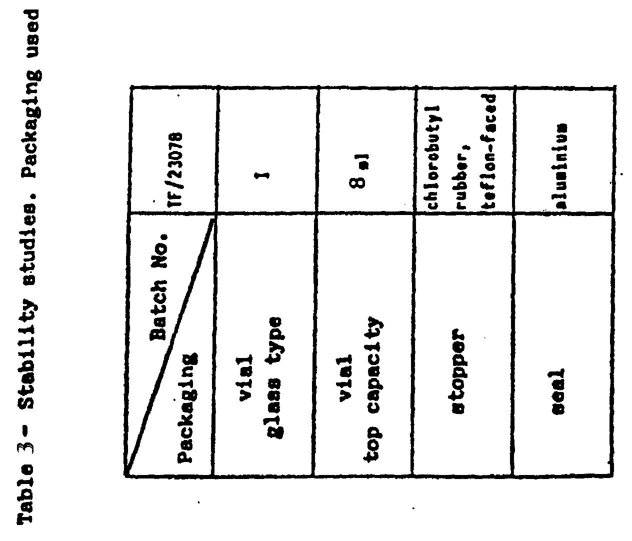

**Table 4 --**

| Doxorubicin·HCl 2 mg/ml solution. Stability data at 4°C (vials stored upright) acquired up to 24 months | | | | | | | | |
|---|---|---|---|---|---|---|---|---|
| Batch Dosage | Time Months | 0 | 1 | 3 | 6 | 9 | 12 | 18 |
| | | | | | | | | |
| TF/23078 | * | 100 | 101.2 | 98.8 | 97.8 | 98.8 | 96.8 | 96.3 |
| 10 mg | ** | 3.50 | 3.54 | 3.49 | 3.44 | 3.43 | 3.54 | 3.50 |

| | | | | | | | | |
|---|---|---|---|---|---|---|---|---|
| * doxorubicin·HCl % stability | | | | | | | | |
| ** pH n.d. = not determined | | | | | | | | |

**Table 5 --**

| Doxoribicin·HCl 2 mg/ml solution. Stability data at 8°C (vials stored upright) acquired up to 24 months. | | | | | | | | |
|---|---|---|---|---|---|---|---|---|
| Batch Dosage | Time Months | 0 | 1 | 3 | 6 | 9 | 12 | 18 |
| TF/23078 | * | 100 | 98.3 | 97.7 | 96.5 | 95.9 | 98.8 | 92.8 |
| 10 mg | ** | 3.50 | 3.59 | 3.47 | 3.27 | 3.43 | 3.51 | 3.40 |

| | | | | | | | | |
|---|---|---|---|---|---|---|---|---|
| * doxorubicin·HCl % stability | | | | | | | | |
| ** pH | | | | | | | | |

**Table 6 --**

| Doxorubicin·HCl 2 mg/ml solution. Stability data at 15°C (vials stored upright) acquired up to 12 months. | | | | | | | | |
|---|---|---|---|---|---|---|---|---|
| Batch Dosage | Time Months | 0 | 0.5 | 1 | 3 | 6 | 9 | 12 |
| TF/23078 | * | 100 | 101.4 | 98.4 | 95.3 | 94.6 | 91.9 | 90.7 |
| 10 mg | ** | 3.50 | 3.51 | 3.58 | 3.47 | 3.38 | 3.41 | 3.47 |

| | | | | | | | | |
|---|---|---|---|---|---|---|---|---|
| * doxorubicin·HCl % stability | | | | | | | | |
| ** pH | | | | | | | | |

**Table 7 --**

| Doxorubicin·HCl 2 mg/ml solution. Stability data at 27°C (vials stored upright) acquired up to 6 months. | | | | | | |
|---|---|---|---|---|---|---|
| Batch Dosage | Time Months | 0 | 0.5 | 1 | 3 | 6 |
| TF/23078 | * | 100 | 101.2 | 94.5 | 80.5 | 71.1 |
| 10 mg | ** | 3.50 | 3.51 | 3.58 | 3.38 | 3.13 |

| | | | | | | |
|---|---|---|---|---|---|---|
| * doxorubicin·HCl % stability | | | | | | |
| ** pH | | | | | | |

**Table 8 --**

| Doxorubicin·HCl 2 mg/ml solution. Stability data at 100 and 250 f.c. (vials stored inverted) acquired up to 3 months | | | | | | | |
|---|---|---|---|---|---|---|---|
| Batch Dosage | Time Months | 100 foot-candles | | | | 250 foot-candles | |
| | | 0 | 0.5 | 1 | 3 | 0.5 | 1 |
| TF/23078 | * | 100 | 99.6 | 96.4 | 88.0 | 97.8 | 89.7 |
| 10 mg | ** | 3.50 | 3.50 | 3.58 | 3.39 | 3.47 | 3.53 |

| | | | | | | | |
|---|---|---|---|---|---|---|---|
| * doxorubicin·HCl % stability | | | | | | | |
| ** pH | | | | | | | |

### Example 2: Long term stability data for doxorubicin.HCl solutions at pH 3.7

A solution of doxorubicin.HCl (2 mg/ml) in sterile water solution and containing 0.9% by weight sodium chloride (batch TF/23256, 10 mg) was prepared. The solution was adjusted to pH 3.7 by 0.1 N hydrochloric acid. The stability data for the solution at different storage temperatures are shown in Table 9 below.

**TABLE 9**

| Storage temperature | Tests | Time (months) | | |
|---|---|---|---|---|
| | | 0 | 1 | 3 |
| 8°C | Doxorubicin.HCl assay (mg/ml) | 2.064 | 2.054 | 2.063 |
| | % stability | 100.0 | 99.3 | 100.0 |
| | pH | 3.7 | 3.7 | 3.6 |
| 15°C | Doxorubicin.HCl assay (mg/ml) | 2.064 | 2.073 | 2.029 |
| | % stability. | 100.0 | 100.5 | 98.4 |
| | pH | 3.7 | 3.7 | 3.6 |
| 27°C | Doxorubicin.HCl assay (mg/ml) | 2.064 | 1.888 | 1.545 |
| | % stability | 100.0 | 91.5 | 74.9 |
| | pH | 3.7 | 3.6 | 3.3 |

## Claims (Claims for the following Contracting State(s): AT, BE, CH, DE, FR, GB, IT, LU, NL, SE)

1. A product which is a storage stable, sterile, pyrogen-free, injectable doxorubicin solution which is sealed in a container and which consists essentially of doxorubicin hydrochloride dissolved in a physiologically acceptable aqueous solvent therefor at a concentration of doxorubicin of from 0.1 to 50 mg/ml, which has not been reconstituted from a lyophilisate and the pH of which has been adjusted to from 3.5 to 4.0 solely by means of a physiologically acceptable acid.

2. A product according to claim 1, wherein the solution has a pH of from 3.7 to 4.0.

3. A product according to claim 1 or 2, wherein the said acid is hydrochloric acid.

4. A product according to any one of the preceding claims, wherein the solution contains dextrose, lactose, sorbitol or mannitol as a tonicity adjustment agent.

5. A product according to any one of the preceding claims, wherein the container is a sealed glass container.

6. A product according to claim 5, wherein the sealed glass container is a vial or an ampule.

7. A process for producing a product according to any one of the preceding claims, which process comprises
(i) dissolving the doxorubicin hydrochloride, which is not in the form of a lyophilisate, in the physiologically acceptable aqueous solvent therefor at a concentration of doxorubicin of from 0.1 to 50 mg/ml;
(ii) optionally, adding one or more formulation adjuvants selected from co-solubilising agents, tonicity adjustment agents, preservatives and pharmaceutically acceptable chelating agents;
(iii) adding solely a physiologically acceptable acid to adjust the pH to from 3.5 to 4.0 as desired; and
(iv) sealing the solution in a container
the process being effected in such a manner that the resultant solution is sterile and pyrogen-free.

8. A process according to claim 7, wherein the solution is passed through a sterilising filter after step (iii).

## Claims (Claims for the following Contracting State(s): ES, GR)

1. A process for producing a product which is a storage stable, sterile, pyrogen-free, injectable solution of doxorubicin sealed in a container, which process comprises
(i) dissolving doxorubicin hydrochloride, which is not in the form of a lyophilisate, in a physiologically acceptable aqueous solvent therefor at a concentration of doxorubicin of from 0.1 to 50 mg/ml;
(ii) optionally, adding one or more formulation adjuvants selected from co-solubilising agents, tonicity adjustment agents, preservatives and pharmaceutically acceptable chelating agents;
(iii) adding solely a physiologically acceptable acid to adjust the pH to from 3.5 to 4.0 as desired; and
(iv) sealing the solution in a container
the process being effected in such a manner that the resultant solution is sterile and pyrogen-free.

2. A process according to claim 1, wherein the solution is passed through a sterilising filter after step (iii).

3. A process according to claim 1 or 2, wherein, in step (iii), the pH is adjusted to from 3.7 to 4.0.

4. A process according to any one of the preceding claims, wherein the said acid is hydrochloric acid.

5. A process according to any one of the preceding claims, wherein dextrose, lactose, sorbitol or mannitol is added in step (iii) as a tonicity adjustment agent.

6. A process according to any one of the preceding claims, wherein the container is a sealed glass container.

7. A process according to claim 6, wherein the sealed glass container is a vial or an ampule.

8. A product obtainable by a process according to any one of the preceding claims.

## Patentansprüche (Patentansprüche für folgende(n) Vertragsstaat(en): AT, BE, CH, DE, FR, GB, IT, LU, NL, SE)

1. Produkt, das eine lagerstabile, sterile, pyrogenfreie, injizierbare Doxorubicin-Lösung ist, die in einem Behälter versiegelt ist und die im wesentlichen aus Doxorubicinhydrochlorid besteht, das in einem physiologisch akzeptablen wäßrigen Lösungsmittel dafür mit einer Doxorubicin-Konzentration von 0,1 bis 50 mg/ml gelöst ist, die nicht aus einem Lyophilisat rekonstituiert wurde, und deren pH-Wert allein mittels einer physiologisch akzeptablen Säure auf 3,5 bis 4,0 eingestellt wurde.

2. Produkt gemäß Anspruch 1, worin die Lösung einen pH-Wert von 3,7 bis 4,0 hat.

3. Produkt gemäß Anspruch 1 oder 2, worin die Säure Salzsäure ist.

4. Produkt gemäß einem der vorhergehenden Ansprüche, worin die Lösung Dextrose, Lactose, Sorbit oder Mannit als Mittel zur Einstellung der Tonizität enthält.

5. Produkt gemäß einem der vorhergehenden Ansprüche, worin der Behälter ein versiegelter Glasbehälter ist.

6. Produkt gemäß Anspruch 5, worin der versiegelte Glasbehälter ein Fläschchen oder eine Ampulle ist.

7. Verfahren zur Herstellung eines Produkts gemäß einem der vorhergehenden Ansprüche, wobei das Verfahren umfaßt:
(i) Auflösen des Doxorubicinhydrochlorids, das nicht in Form eines Lyophilisats ist, in dem physiologisch akzeptablen wäßrigen Lösungsmittel dafür mit einer Doxorubicin-Konzentration von 0,1 bis 50 mg/ml;
(ii) gegebenenfalls Zugeben eines oder mehrerer Formulierungsadjuvantien, die aus Co-Solubilisierungsmitteln, Mitteln zur Einstellung der Tonizität, Konservierungsmitteln und pharmazeutisch akzeptablen Komplexbildnern ausgewählt sind;
(iii) Zugeben allein einer physiologisch akzeptablen Säure zur Einstellung des pH-Wertes auf 3,5 bis 4,0 nach Wunsch; und
(iv) Versiegeln der Lösung in einem Behälter,
wobei das Verfahren in einer solchen Weise bewirkt wird, daß die resultierende Lösung steril und pyrogenfrei ist.

8. Verfahren gemäß Anspruch 7, worin die Lösung durch einen sterilisierenden Filter nach Schritt (iii) geleitet wird.

## Patentansprüche (Patentansprüche für folgende(n) Vertragsstaat(en): ES, GR)

1. Verfahren zur Herstellung eines Produkts, das eine lagerstabile, sterile, pyrogenfreie, injizierbare Doxorubicin-Lösung ist, die in einem Behälter versiegelt ist, wobei das Verfahren umfaßt:
(i) Auflösen von Doxorubicinhydrochlorid, das nicht in Form eines Lyophilisats ist, in einem physiologisch akzeptablen wäßrigen Lösungsmittel dafür mit einer Doxorubicin-Konzentration von 0,1 bis 50 mg/ml;
(ii) gegebenenfalls Zugeben eines oder mehrerer Formulierungsadjuvantien, die aus Co-Solubilisierungsmitteln, Mitteln zur Einstellung der Tonizität, Konservierungsmitteln und pharmazeutisch akzeptablen Komplexbildnern ausgewählt sind;
(iii) Zugeben allein einer physiologisch akzeptablen Säure zur Einstellung des pH-Wertes auf 3,5 bis 4,0 nach Wunsch; und
(iv) Versiegeln der Lösung in einem Behälter,
wobei das Verfahren in einer solchen Weise bewirkt wird, daß die resultierende Lösung steril und pyrogenfrei ist.

2. Verfahren gemäß Anspruch 1, worin die Lösung durch einen sterilisierenden Filter nach Schritt (iii) geleitet wird.

3. Verfahren gemäß Anspruch 1 oder 2, worin in Schritt (iii) der pH-Wert auf 3,7 bis 4,0 eingestellt wird.

4. Verfahren gemäß einem der vorhergehenden Ansprüche, worin die Säure Salzsäure ist.

5. Verfahren gemäß einem der vorhergehenden Ansprüche, worin Dextrose, Lactose, Sorbit oder Mannit in Schritt (iii) als Mittel zur Einstellung der Tonizität zugegeben wird.

6. Verfahren gemäß einem der vorhergehenden Ansprüche, worin der Behälter ein versiegelter Glasbehälter ist.

7. Verfahren gemäß Anspruch 6, worin der versiegelte Glasbehälter ein Fläschchen oder eine Ampulle ist.

8. Produkt, das durch ein Verfahren gemäß einem der vorhergehenden Ansprüche erhältlich ist.

## Revendications (Revendications pour l'(les) Etat(s) contractant(s) suivant(s): AT, BE, CH, DE, FR, GB, IT, LU, NL, SE)

1. Produit qui est une solution de doxorubicine stable au stockage, stérile, exempte de pyrogène, injectable, scellée dans un récipient et constituée essentiellement de chlorhydrate de doxorubicine dissous dans un solvant aqueux physiologiquement acceptable pour celui-ci à une concentration de doxorubicine de 0,1 à 50 mg/ml, qui n'a pas été reconstitué à partir d'un lyophilisat et dont le pH a été ajusté à 3,5-4,0 uniquement au moyen d'un acide physiologiquement acceptable.

2. Produit selon la revendication 1, dont la solution a un pH de 3,7 à 4,0.

3. Produit selon la revendication 1 ou 2, pour lequel ledit acide est l'acide chlorhydrique.

4. Produit selon l'une quelconque des revendications précédentes, dont la solution contient du dextrose, du lactose, du sorbitol ou du mannitol en tant qu'agent d'ajustement de la tonicité.

5. Produit selon l'une quelconque des revendications précédentes, dont le récipient est un récipient en verre scellé.

6. Produit selon la revendication 5, dont le récipient en verre scellé est une fiole ou une ampoule.

7. Procédé pour produire un produit selon l'une quelconque des revendications précédentes, lequel procédé comprend
(i) la dissolution du chlorhydrate de doxorubicine, qui n'est pas sous la forme d'un lyophilisat, dans un solvant aqueux physiologiquement acceptable pour celui-ci à une concentration de doxorubicine de 0,1 à 50 mg/ml ;
(ii) éventuellement, l'addition d'un ou plusieurs adjuvant(s)de formulation choisi(s) parmi des agents de co-solubilisation, des agents d'ajustement de la tonicité, des conservateurs et des agents chélatants pharmaceutiquement acceptables ;
(iii)l'addition uniquement d'un acide physiologiquement acceptable pour ajuster le pH à 3,5-4,0 si souhaité ; et
(iv) le scellement de la solution dans un récipient,
le procédé étant mis en oeuvre de telle manière que la solution résultante soit stérile et exempte de pyrogène.

8. Procédé selon la revendication 7, dans lequel la solution est passée à travers un filtre stérilisant après l'étape (iii).

## Revendications (Revendications pour l'(les) Etat(s) contractant(s) suivant(s): ES, GR)

1. Procédé pour produire un produit qui est une solution de doxorubicine stable au stockage, stérile, exempte de pyrogène, injectable, scellée dans un récipient, lequel procédé comprend
(i) la dissolution de chlorhydrate de doxorubicine, qui n'est pas sous la forme d'un lyophilisat, dans un solvant aqueux physiologiquement acceptable pour celui-ci à une concentration de 0,1 à 50 mg/ml ;
(ii) éventuellement, l'addition d'un ou plusieurs adjuvant(s) de formulation(s) choisi(s) parmi des agents de co-solubilisation, des agents d'ajustement de la tonicité, des conservateurs et des agents chélatants pharmaceutiquement acceptables ;
(iii)l'addition uniquement d'un acide physiologiquement acceptable pour ajuster le pH de 3,5-4,0 si souhaité ; et
(iv) le scellement de la solution dans un récipient,
le procédé étant mis en oeuvre de telle manière que la solution résultante soit stérile et exempte de pyrogène.

2. Procédé selon la revendication 1, dans lequel la solution est passée à travers un filtre stérilisant après l'étape (iii).

3. Procédé selon la revendication 1 ou 2, dans lequel, dans l'étape (iii), le pH est ajusté à 3,7-4,0.

4. Procédé selon l'une quelconque des revendications précédentes, dans lequel ledit acide est l'acide chlorhydrique.

5. Procédé selon l'une quelconque des revendications précédentes, dans lequel on ajoute à l'étape (iii) du dextrose, du lactose, du sorbitol ou du mannitol en tant qu'agent d'ajustement de la tonicité.

6. Procédé selon l'une quelconque des revendications précédentes, dans lequel le récipient est un récipient en verre scellé.

7. Procédé selon la revendication 6, dans lequel le réservoir en verre scellé est une fiole ou une ampoule.

8. Produit que l'on peut obtenir par un procédé selon l'une quelconque des revendications précédentes.
